# EUROPEAN PATENT APPLICATION

(11) **EP 3 192 459 A1**
(43) Date of publication of application: **19.07.2017**
(21) Application number: 17157774.5
(22) Date of filing: 13.06.2013
(51) Int. Cl.: A61B 17/072, A61B 17/29

(54) **SLIDING ANVIL/RETRACTING CARTRIDGE RELOAD**

(30) Priority: 14.06.2012 US 201261659567 P; 05.04.2013 US 201313857202
(62) Divisional of application: 13171803.3
(71) Applicant: Covidien LP, Mansfield, MA 02048 (US)
(72) Inventor: Krehel, Gregg, Newtown, Connecticut 06470 (US); Cabrera, Ramiro, Cheshire, Connecticut 06410 (US); Whitfield, Kenneth, North Haven, Connecticut 06473 (US); Racenet, Danyel, Killingworth, Connecticut 06419 (US)
(74) Representative: Maschio & Soames IP Ltd

(57) **Abstract**

A surgical device comprising: a handle assembly (12); an elongated member (18) extending distally from the handle assembly, the elongated member defining a longitudinal axis; a tool assembly (104) mounted to a distal end of the elongated member, the tool assembly having a cartridge assembly (108) having a plurality of staples (130) supported therein and an anvil assembly (206), at least one of the anvil assembly and the cartridge assembly being movable in relation to each other between open and closed positions, the anvil assembly having a portion thereof being configured for transitioning between a longitudinally extended state and a longitudinally retracted state; and a transitioning mechanism configured to transition the anvil assembly (206) between the extended and retracted states, characterized in that: said portion configured for transitioning between a longitudinally extended state and a longitudinally retracted state comprises a compliant pneumatic tip (250) having a rolled configuration in the retracted state and an unrolled configuration in the extended state, the pneumatic tip being biased to the retracted state; and said transitioning mechanism comprises a translation rod (154), the pneumatic tip (250) and the translation rod being hollow for receipt of a pressurized fluid therethrough, whereby application of a fluid pressure through the hollow translation rod (154) extends the pneumatic tip (250) and removal of the fluid pressure retracts the pneumatic tip (250).

## Description

### CROSS-REFERENCE TO RELATED APPLICATIONS

The present application claims priority to, and the benefit of, U.S. Provisional Patent Application Serial No. 61/659,567, filed on June 14, 2012, the entire contents of which are hereby incorporated by reference.

### BACKGROUND

### 1. Technical Field

The present disclosure relates to a surgical stapling device and, more particularly, to an endoscopic surgical stapling device configured to operate a tool assembly with an extendable tip element for manipulating tissue that is usable in thoracic procedures.

### 2. Background of Related Art

Surgical devices wherein tissue is first grasped or clamped between opposing jaw structures and then joined by surgical fasteners are well known in the art. The fasteners are typically in the form of surgical staples, but two-part polymeric fasteners can also be utilized.

Instruments for this purpose can include two elongated members which are respectively used to capture or clamp tissue. Typically, one of the members carries a staple cartridge which houses a plurality of staples arranged, for example, in at least two lateral rows while the other member has an anvil that defines a surface for forming the staple legs as the staples are driven from the staple cartridge. Generally, the stapling operation is effected by sleds that travel longitudinally through the staple cartridge, with the sleds acting upon staple pushers to sequentially eject the staples from the staple cartridge. A knife can travel between the staple rows to longitudinally cut the stapled tissue between the rows of staples. Such staplers are disclosed in U.S. Patent Nos. 6,250,532 and 6,241,139, each of which are currently owned by Tyco Healthcare Group LP, and are incorporated herein by reference in their entirety.

In minimally invasive thoracic procedures, surgery is performed through small incisions or through small diameter cannulas or seal anchors inserted through small entrance wounds in the skin. During minimally invasive thoracic procedures in particular, surgery is performed through the natural intercostal space between adjacent ribs. Due to the limited degree of motion of an instrument when it is positioned between the ribs, it may be quite difficult for a surgeon to manipulate the tool assembly of the instrument around body tissue extraneous to the procedure to access and/or clamp the tissue site. Instruments having rotatable endoscopic body portions and rotatable and/or articulatable tool assemblies have been developed to overcome this problem and are commercially available. Although these instruments provide significant improvements in the endoscopic tool art, further improvements that may decrease the time required for surgical procedures by allowing surgeons to more easily access tissue sites are desired. Accordingly, a continuing need exists for a minimally invasive surgical device having a tool assembly that can quickly and easily manipulate tissue.

### SUMMARY

In one aspect, a surgical device is provided having a handle assembly and an elongated member extending distally from the handle assembly and defining a longitudinal axis. A tool assembly is mounted to a distal end of the elongated member. The tool assembly has a cartridge assembly having a plurality of staples. The tool assembly further has an anvil assembly. At least one of the anvil assembly and the cartridge assembly is movable in relation to each other between open and closed positions. At least one of the anvil assembly and the cartridge assembly has at least a portion configured for transitioning between a longitudinally extended state and a longitudinally retracted state. A transitioning mechanism is configured to transition at least one of the anvil assembly and the cartridge assembly between the extended and retracted states.

At least one of the cartridge assembly and the anvil assembly may include a tongue that transitions between an extended state and a retracted state. The transitioning mechanism may pneumatically transition the tongue. The transitioning mechanism may include a rod for transitioning the tongue. The tongue may have an arcuate configuration. The tongue may transition out from and into at least one of the cartridge assembly and the anvil assembly. In some embodiments, the tongue is composed of shape memory material and returns to a shape memorized position in the extended state.

In some embodiments, at least one of the cartridge assembly and the anvil assembly may include a visual aid. The visual aid may be an LED. At least one of the cartridge assembly and the anvil assembly may include a sensor for sensing a tissue condition. At least one of the cartridge assembly and the anvil assembly may include a tube configured for passage of material.

In some embodiments, at least one of the cartridge assembly and the anvil assembly distal to an engagement plane may transition between the expanded state and the contracted state. At least one of the cartridge assembly and the anvil assembly proximal to an engagement plane may remain stationary as at least one of the cartridge assembly and the anvil assembly distal to an engagement plane transitions between the expanded state and the contracted state.

In some embodiments, at least one of the cartridge assembly and the anvil assembly proximal to an engagement plane may transition between the expanded state and the contracted state. At least one of the cartridge assembly and the anvil assembly distal to an engagement plane may remain stationary as at least one of the cartridge assembly and the anvil assembly proximal to an engagement plane transitions between the expanded state and the contracted state.

The cartridge assembly and the anvil assembly may move between the open and closed positions in response to actuation of a movable handle of the handle assembly. The cartridge assembly may fire the plurality of staples in response to actuation of a movable handle of the handle assembly. The surgical device may include a mode selection mechanism configured to alternate the surgical device between a first mode of operation and a second mode of operation. The mode selection mechanism may include a switch for selecting between the first mode of operation, in which the cartridge assembly and anvil assembly are movable back and forth between the open and closed positions, and the second mode of operation, in which the cartridge assembly and anvil assembly are locked in the closed position for clamping tissue and firing the plurality of staples.

In another aspect, a method of stapling tissue is provided having the steps of inserting a surgical stapling device including a end effector having an extendable tip through an incision in tissue, extending the extendable tip, manipulating tissue with the extendable tip, grasping tissue within the end effector, switching the surgical stapling device from a grasping mode to a firing mode, and firing staples from the end effector through tissue. The method may further include the step of pulling a retraction handle of the surgical stapling device to release tissue from within the end effector and return the surgical stapling device from the firing mode to the grasping mode. The extendable tip may be movable out from and into an anvil assembly or cartridge assembly of the end effector. The extendable tip may be curved tongue.

### BRIEF DESCRIPTION OF THE DRAWINGS

Embodiments of the presently disclosed surgical stapling device are disclosed herein with reference to the drawings wherein:

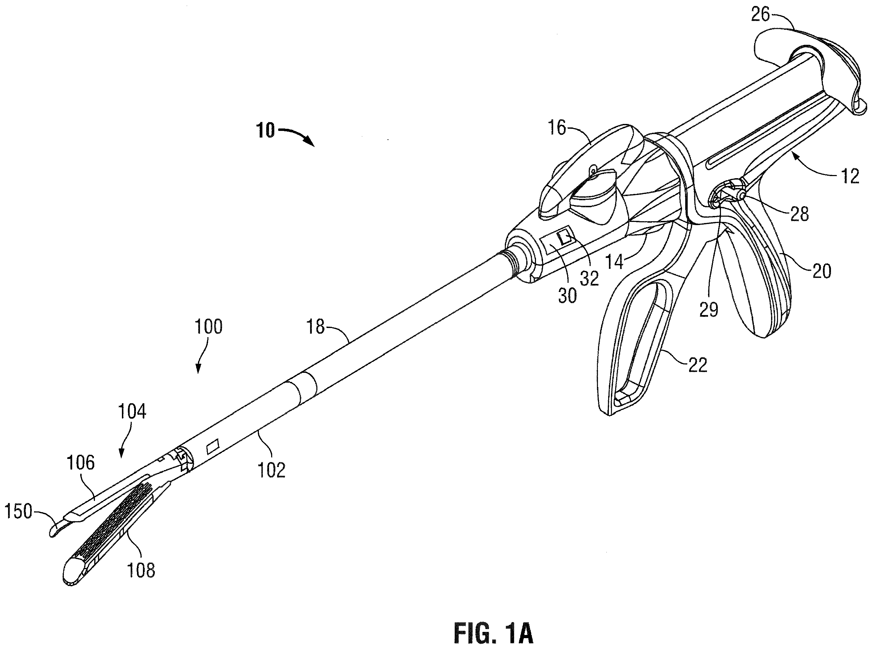

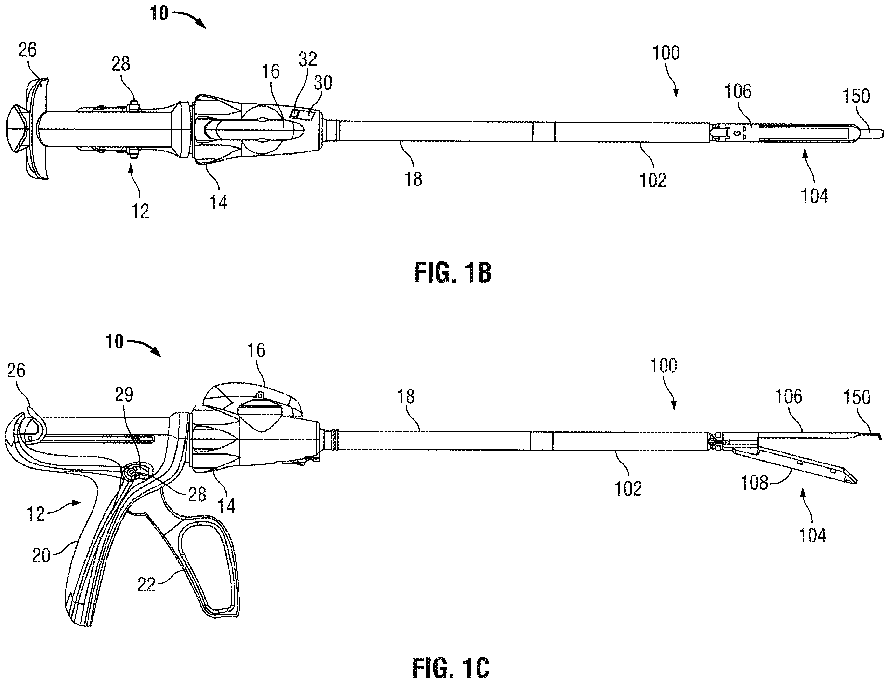

**FIG. 1A** is a side perspective view of a surgical stapling device according to the present disclosure;
**FIG. 1** **B** is a top view illustrating the surgical stapling device of **FIG. 1A****;**
**FIG. 1C** is a side view illustrating the surgical stapling device of **FIG. 1A****;**
**FIG. 2A** is an exploded view of a handle assembly of the surgical stapling device of **FIG. 1A****;**
**FIG. 2B** is a side, perspective view of an actuation bar of the handle assembly of **FIG. 2A****;**
**FIG. 2C** is a side, cross-sectional view of the actuation bar of **FIG. 2B**;
**FIG. 3A** is a side, cross-section view of the handle assembly of **FIG. 2A** with a movable handle thereof in an first position;
**FIG. 3B** is a side, cross-sectional view of a pawl assembly of the handle assembly of **FIG. 2A****;**
**FIG. 3C** is a side, cross-sectional view of the pawl assembly of **FIG. 3B** with the movable handle in a second position;
**FIG. 3D** is a side, perspective view of the handle mechanism of **FIG. 2A** being transitioned from a grasping mode to a firing mode;
**FIG. 3E** is a side, cross-sectional view of the pawl assembly of **FIG. 3B** in the firing mode while the movable handle is in the first position;
**FIG. 3F** is a side, perspective view of the pawl assembly of **FIG. 3B****;**
**FIG. 3G** is a side perspective view of the pawl assembly of **FIG. 3F** in the firing mode as the movable handle is returning to the second position;
**FIG. 3H** is a side, cross-sectional view of the pawl assembly of **FIG. 3G****;**
**FIG. 3I** is a side, cross-sectional view of the pawl assembly of **FIG. 3H** as the movable handle is pulled to the first position while in firing mode;
**FIG. 4** is a side perspective view illustrating an end effector of the surgical stapling device of **FIG. 1A** in an open position with a tongue extended therefrom;
**FIG. 5** is a front cross-sectional view of the end effector of **FIG. 4**;
**FIG. 6A** is a side cross-sectional view of a tool assembly of the end effector of FIG. 4 in a closed position with the tongue retracted therein;
**FIG. 6B** is a side cross-sectional view of the tool assembly of **FIG. 6A** in an open position with the tongue extended therefrom;
**FIG. 7** is a side cross-sectional view of the tool assembly of **FIG. 6A** illustrating distal translation of a drive member and a sled;
**FIG. 8** is a side cross-sectional view of the tool assembly of **FIG. 6A** illustrating firing of a plurality of staples;
**FIG. 9A** is a side, cross-sectional view of the surgical stapling device of **FIG. 1A** prior to entry into an opening in thoracic tissue in a minimally invasive thoracic procedure;
**FIG. 9B** is a side, cross-sectional view of the surgical stapling device of **FIG. 1A** inserted through the opening in thoracic tissue and manipulating a vessel therein;
**FIG. 9C** is a side, cross-sectional view of the surgical stapling device of **FIG. 1A** stapling the vessel;
**FIG. 10** is a side perspective view of an anvil assembly of a surgical stapling device according to an embodiment of the present disclosure having a pneumatically controlled tongue;
**FIG. 11** is a side view of a tool assembly of a surgical stapling device according to an embodiment of the present disclosure having a retractable cartridge assembly; and
**FIG. 12** is a side view of an end effector of a surgical stapling device according to an embodiment of the present disclosure having an extendable body portion, an LED tip, and a tube.

### DETAILED DESCRIPTION OF THE EMBODIMENTS

Embodiments of the presently disclosed surgical stapling device will now be described in detail with reference to the drawings in which like reference numerals designate identical or corresponding element in each of the several views.

Throughout this description, the term "proximal" will refer to the portion of the device closer to the operator and the term "distal" will refer to the portion of the device further from the operator.

**FIGS. 1A-1C** illustrate one embodiment of the presently disclosed surgical stapling device shown generally as **10.** Surgical stapling device **10** includes a handle assembly **12,** a rotation knob **14,** an articulation lever **16,** an elongated shaft **18,** and an end effector **100.** Handle assembly **12** includes a stationary handle **20,** a movable handle **22,** and retraction handle **26.** Elongated shaft **18** defines a longitudinal axis of surgical stapling device **10.** An actuator button **28** extends transversely through and projects outwardly from button paths **29** on opposite sides of handle assembly **12.** Actuator button **28** is slidable along button paths **29.** A translation switch **32** is slidable within a switch depression **30** on handle assembly **12.** Surgical stapling device **10** is transitionable between a grasping mode, in which end effector **100** may grasp and release tissue, and a firing mode, in which end effector **100** fires staples through tissue.

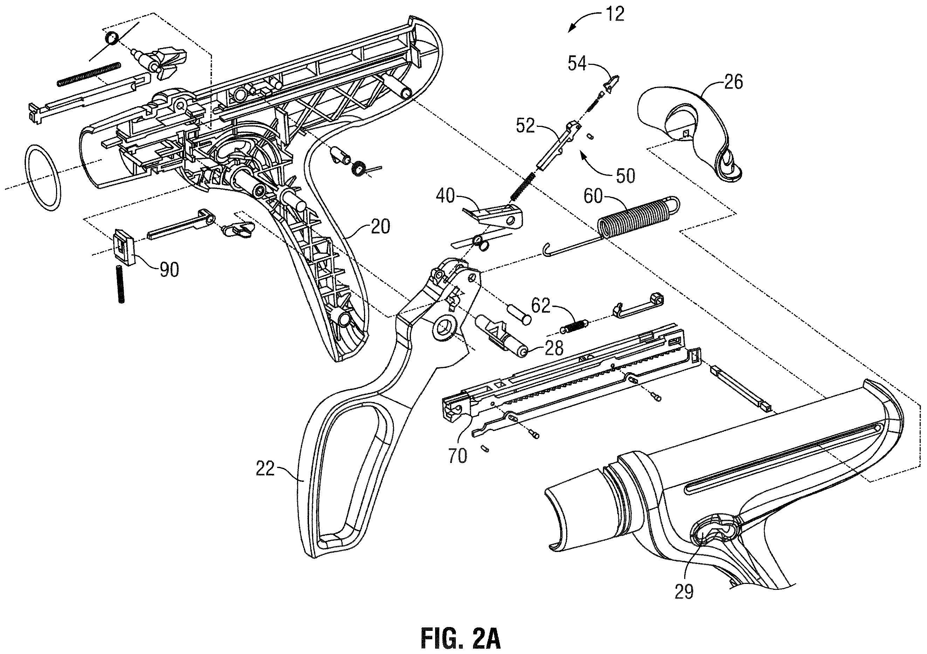

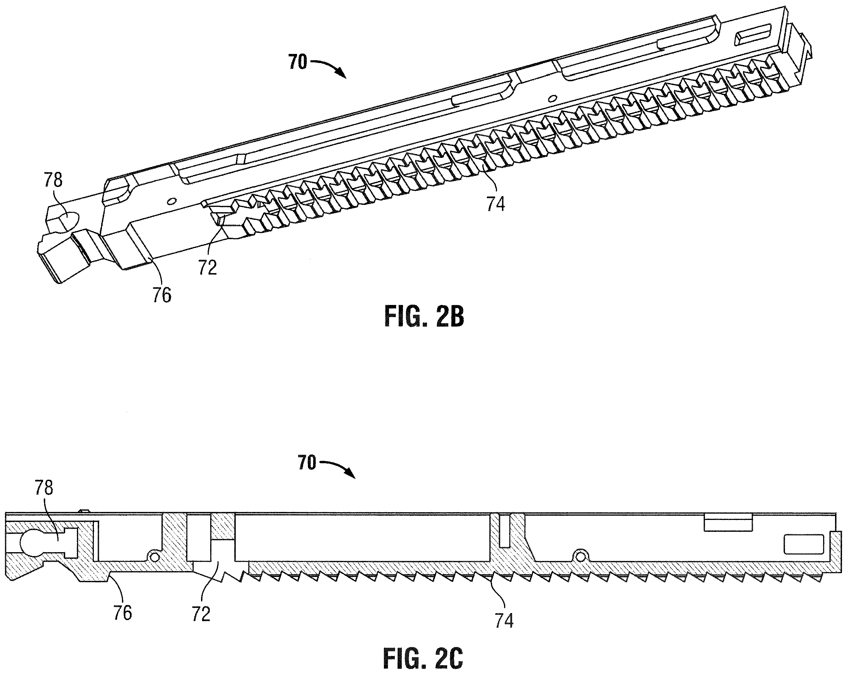

Movable handle **22** pivotably transitions between a first position and a second position. Movable handle **22** is biased by a handle spring **60** (**FIG. 2A**) toward the second position, in which movable handle **22** is at a farthest distance from stationary handle **20.** Pulling movable handle **22** toward stationary handle **20** transitions movable handle **22** to the first position, in which movable handle **22** is at a closest distance to stationary handle **20.** Releasing movable handle **22** allows biasing forces within handle assembly **12** to transition movable handle **22** back to the second position.

End effector **100** includes a body portion **102** and a tool assembly **104** pivotably attached to body portion **102.** Rotation knob **14** may be rotated to actuate rotation of end effector **100** about the longitudinal axis. Rotation of rotation knob **14** may rotate elongated shaft **18** and end effector **100.** Articulation lever **16** may be turned to actuate articulation of tool assembly **104,** which moves tool assembly **104** off axis relative to the longitudinal axis. In some embodiments, end effector **100** may be a disposable loading unit (DLU) that is releasably attached to elongated shaft **18.**

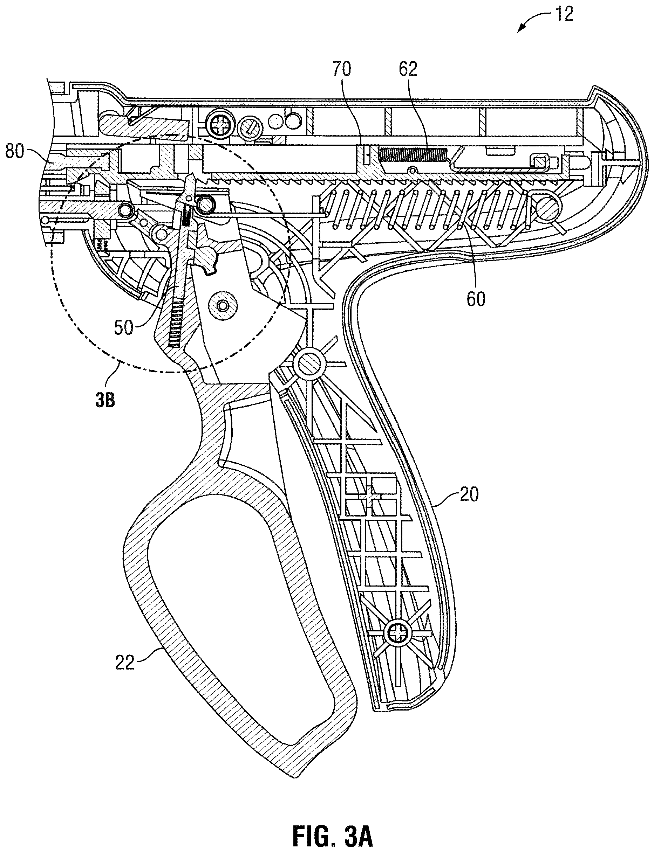

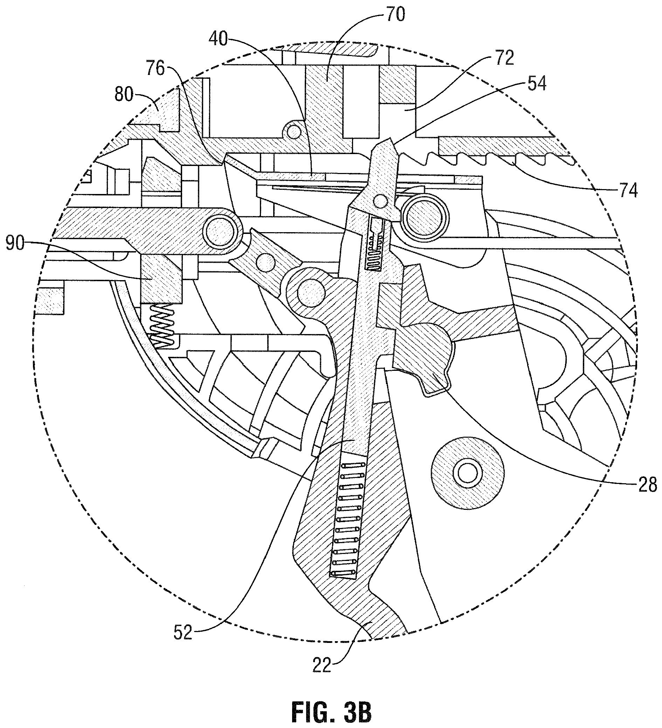

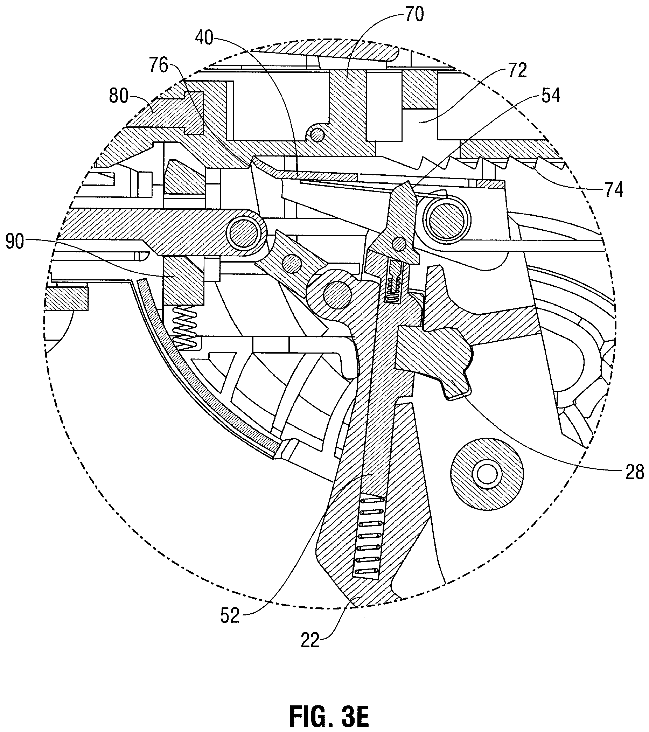

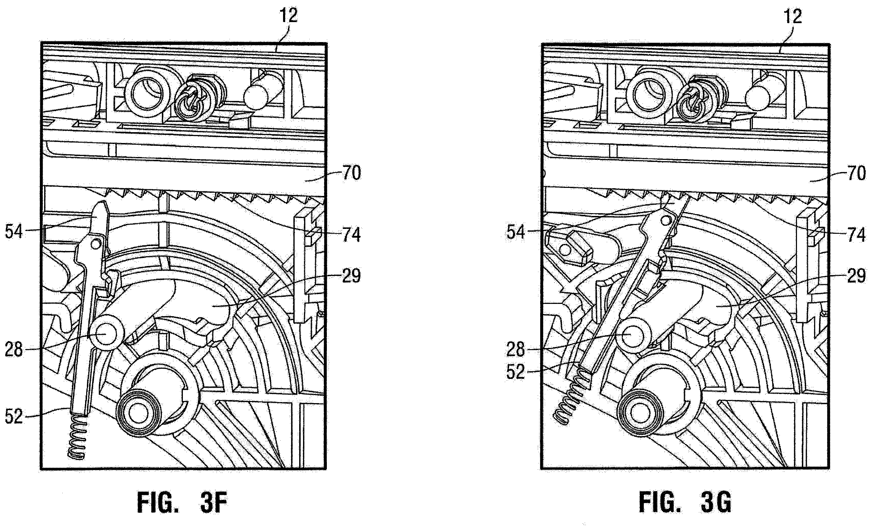

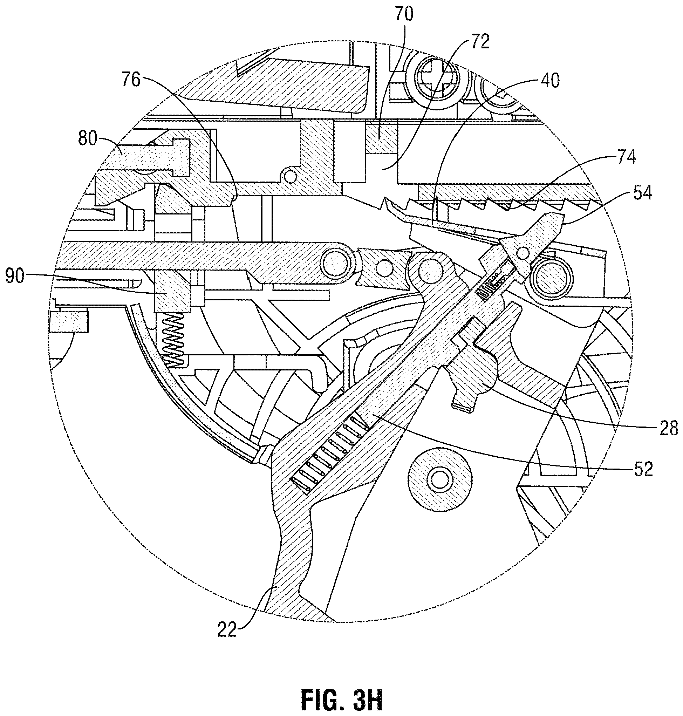

As seen in **FIG. 2A****,** handle assembly **12** includes a pawl assembly **50.** Pawl assembly **50** includes a pawl arm **52** and a grasping pawl **54.** Handle assembly **12** further includes a driving pawl **40** and a vertical pawl **90.** Handle assembly **12** further includes handle spring **60** for biasing movable handle **22** toward the second position and an actuator spring **62** for biasing an actuator bar **70** proximally. Actuator bar **70** translates an actuation rod **80** (**FIG. 3A**) for actuating grasping and firing of tool assembly **104.** Pawl arm **52** is operably connected to actuator button **28** such that a distal sliding of actuator button **28** retracts pawl assembly **50** as will be discussed in greater detail hereinbelow.

As seen in **FIGS. 2B-2C****,** actuator bar **70** has a pawl gap **72,** teeth **74,** an abutment **76,** and a rod recess **78.** Rod recess **78** is configured and dimensioned to receive a proximal end of actuation rod **80** therein. Pawl gap **72** is configured and dimensioned to removably receive pawl **54** therein when surgical stapling device **10** is in the grasping mode. Abutment **76** is configured to engage driving pawl **42** to drive actuation bar **70** distally in the grasping mode. Teeth **74** are configured to engage driving pawl **42** to drive actuation bar **70** distally in the firing mode.

**FIGS. 3A-3I** illustrate pawl assembly **50** as surgical stapling device **10** is transitioned from the grasping mode to the firing mode. As seen in **FIGS. 3A-3B****,** surgical stapling device **10** begins in the grasping mode. Pulling movable handle **22** from the second position to the first position urges driving pawl **40** distally. Driving pawl **40** contacts abutment **76** to drive actuator bar **70** distally. Distal translation of actuator bar **70** distally translates actuation rod **80,** thereby causing end effector **100** to grasp tissue, as will be explained in greater detail hereinbelow. Grasping pawl **54** remains in pawl gap **72** in the grasping mode.

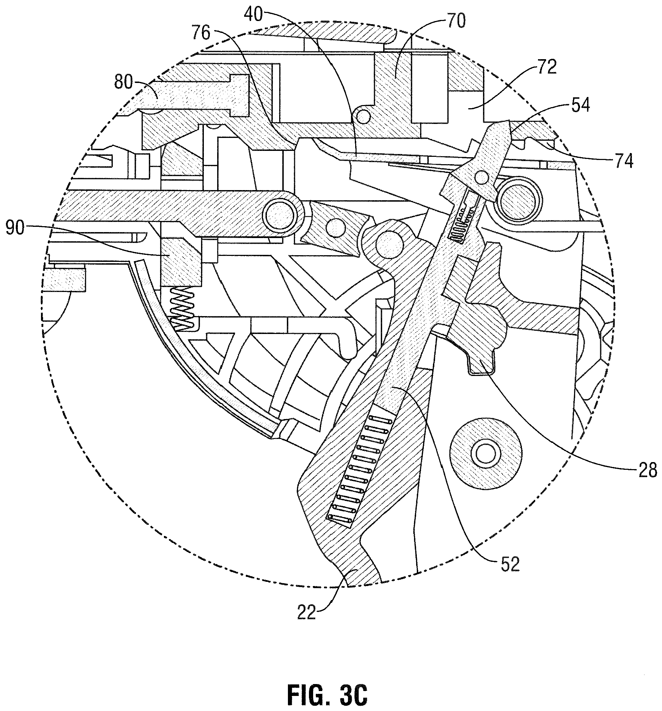

**FIG. 3C** illustrates pawl assembly **50** as movable handle **22** is released back to the second position. Driving pawl **40** retreats proximally from abutment **76** and thus no longer urges actuator bar **70** distally. Actuator spring **62** translates actuator bar **70** proximally, which in turn pulls actuation rod **80** proximally and causes end effector **100** to release its grasp on tissue.

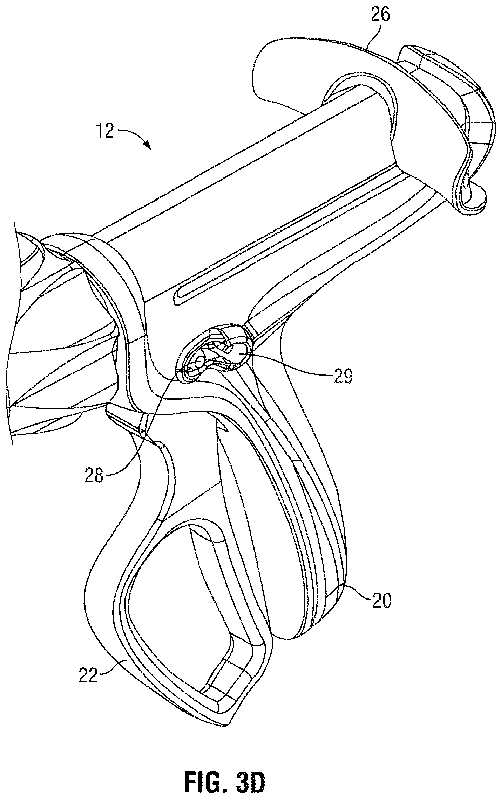

As seen in **FIGS. 3D-3F****,** when surgical stapling device **10** is in the grasping mode and movable handle **22** is moved to the first position, actuator button **28** can be slid distally to transition surgical stapling device **10** from the grasping mode to the firing mode. Sliding actuator button **28** distally retracts grasping pawl **54** from pawl gap **72.**

As seen in **FIGS. 3G-3H****,** when movable handle **22** is released for the first time after surgical stapling device **10** is transitioned to the firing mode, pawl arm **52** rotates such that grasping pawl **54,** which is no longer within pawl gap **72** and thus not inhibited from moving proximally, is moved to a position proximal to pawl gap **72.** As pawl arm **52** rotates, pawl arm 52 slides actuator button **28** proximally, thereby returning pawl assembly **50** from the retracted position. Driving pawl **40** now engages teeth **74.**

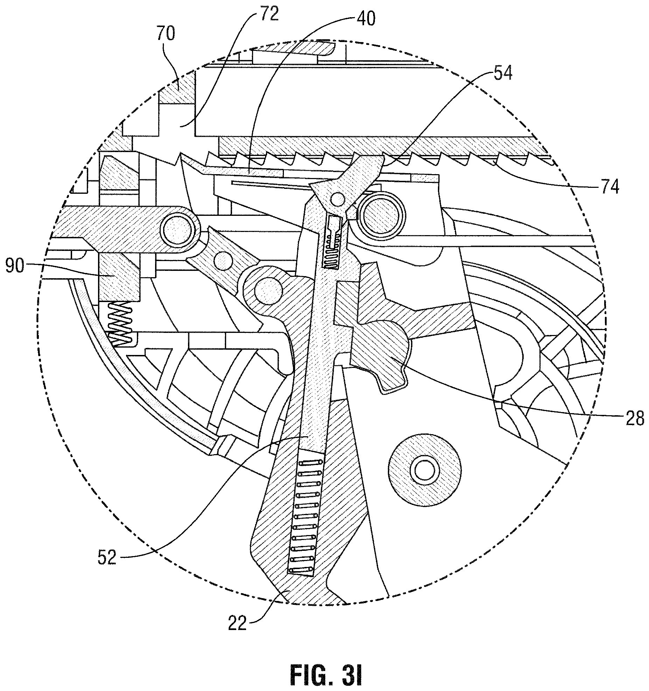

As seen in **FIG. 3I****,** an additional pulling of movable handle **22** to the first position drives actuator bar **70** distally. Driving pawl **40** drives actuator bar **70** distally when movable handle **22** is pulled to the first position. Vertical pawl **90** engages actuator bar **70** to inhibit actuator bar **70** from being pulled proximally by actuator spring **62** as movable handle **22** is released to the second position. Grasping pawl **54** and driving pawl **40** slide proximally along teeth **74** as movable handle **22** is released to the second position. Thus, additional pulling and releasing of movable handle **22** incrementally drives actuator bar **70** distally to fire staples. When firing is complete, retraction handle **26** is pulled proximally, which removes actuator bar from contact with driving pawl **40** and grasping pawl **54** and pulls actuator bar **70** proximally. Surgical stapling device **10** is thus returned to the grasping mode.

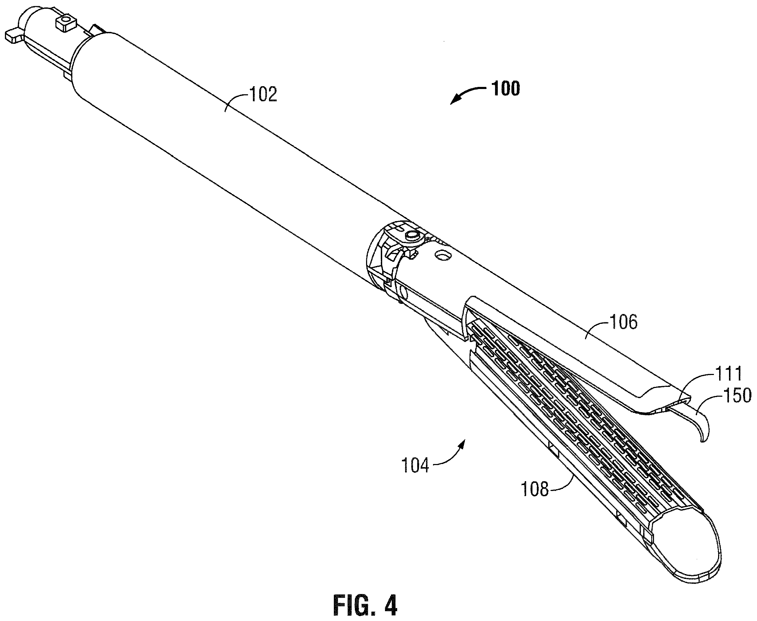

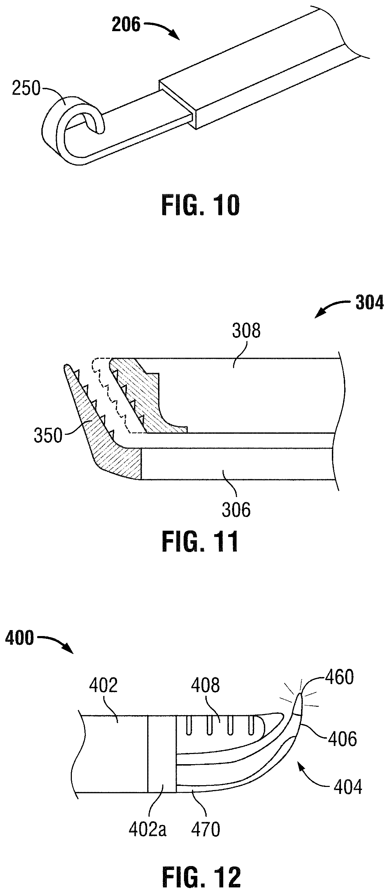

As seen in **FIG. 4****,** tool assembly **104** includes an anvil assembly **106** and a cartridge assembly **108.** Anvil assembly **106** and cartridge assembly **108** are movable back and forth between an open position and a closed position when surgical stapling device **10** is in a grasping mode. Anvil assembly **106** and cartridge assembly **108** are pivotably movable with respect to each other. In one embodiment, anvil assembly **106** and cartridge assembly 108 are also translatably movable with respect to each other (**FIG. 11**). In the open position, anvil assembly **106** and cartridge assembly **108** as spaced sufficiently far apart to receive a tissue therebetween. In the closed position, anvil assembly **106** and cartridge assembly **108** are spaced sufficiently close together to grasp tissue therebetween, *i.e.,* close cooperative alignment. Anvil assembly **106** and cartridge assembly **108** may be substantially parallel in the closed position.

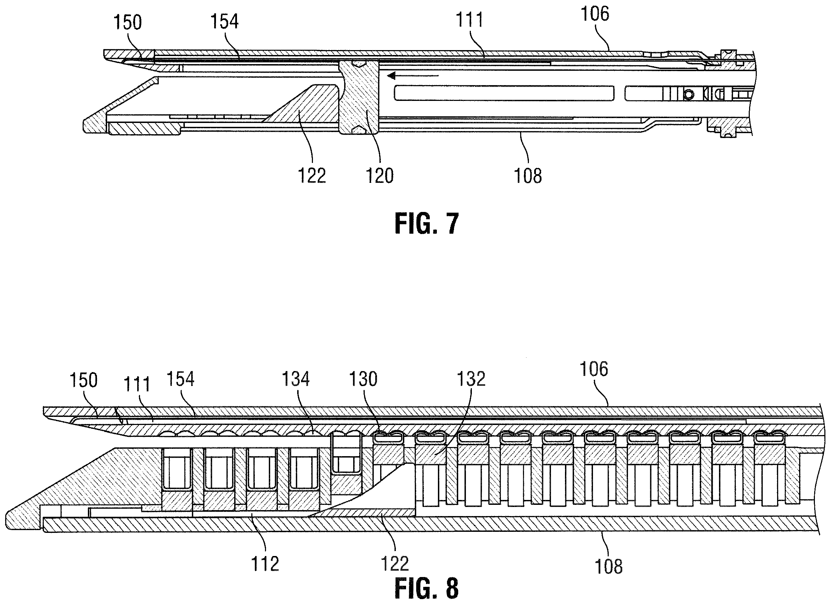

In the grasping mode, anvil assembly **106** and cartridge assembly **108** move to the closed position upon a pulling of movable handle **22** to the first position. Anvil assembly **106** and cartridge assembly **108** return to the open position as movable handle **22** is released to the second position. When tool assembly **104** is in the closed position, surgical stapling device **10** may be transitioned from the grasping mode to a firing mode by sliding actuator button **28** distally. In the firing mode, anvil assembly **106** and cartridge assembly **108** remain in the closed position as movable handle **22** is moved away from stationary handle **20.** Pulling movable handle **22** to the first position once more fires staples **130** (**FIG. 8**) from cartridge assembly **108.** Compression of staples **130** between cartridge assembly **108** and anvil assembly **106** deforms staples **130,** thereby stapling tissue between cartridge assembly **108** and anvil assembly **106.** Surgical stapling device **10** may be returned from the firing mode to the grasping mode by pulling retraction handles **26** proximally. Such a stapler is disclosed in U.S. Patent App. Pub. No. 2011/0272448, which is currently owned by Tyco Healthcare Group LP, and is incorporated herein by reference in their entirety.

Anvil assembly **106** includes a translatable tip **150** for selectively manipulating tissue in a surgical site. In some embodiments, cartridge assembly **108** rather than anvil assembly **106** includes a translatable tip. Embodiments are also conceived in which both anvil assembly **106** and cartridge assembly **108** include translatable tips. Translatable tip **150** is retractable within an anvil channel **111** within anvil assembly **106** upon a sliding of translation switch **32** proximally. Translatable tip **150** is extendable from anvil channel **111** upon a sliding of translation switch **32** distally. Extension and retraction of translatable tip **150** may be partial. Alternatively, translatable tip **150** may be transitioned only between a completely extended state and a completely retracted state.

Translatable tip or tongue **150** may have a generally curved configuration. Translatable tip **150** may be composed of a resiliently deformable material, such as a memory metal, such that translatable tip **150** has a substantially arcuate configuration when extended out of anvil channel **111** and a substantially linear configuration when retracted into anvil channel **111.** Translatable tip **150** may have a soft durometer to assist in transitioning between the retracted and extended states. Translatable tip **150** may have any suitable curvature, for example, translatable tip **150** may curve toward cartridge assembly **108** or away from cartridge assembly **108.** Embodiments are conceived in which translatable tip **150** may be rotated to curve in any desirable direction. Embodiments are also conceived in which translatable tip **150** has adjustable curvature.

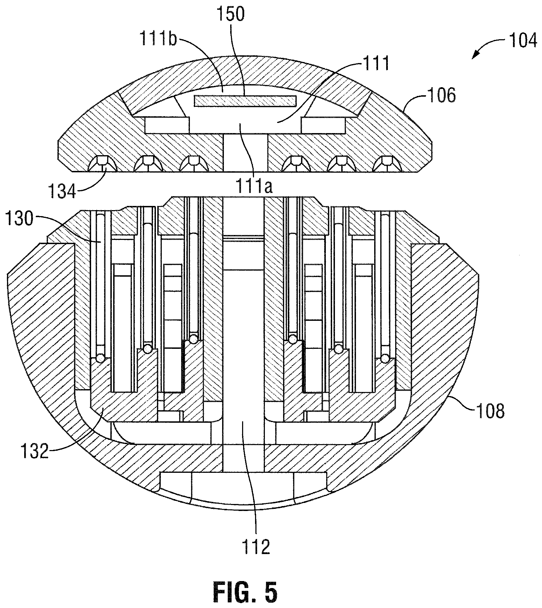

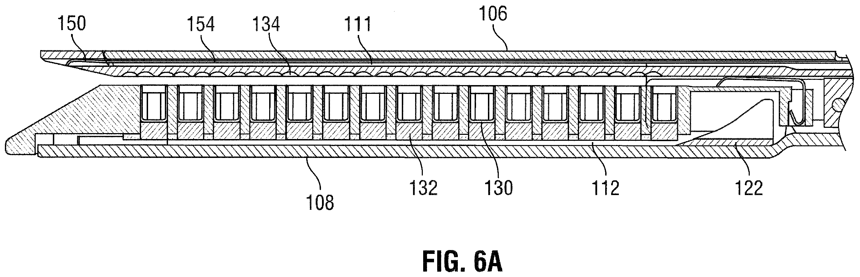

As seen in **FIG. 5****,** anvil assembly **106** includes anvil channel **111** and cartridge assembly includes a cartridge channel **112.** Channels **111, 112** are configured for passage of a drive member **120** (**FIG. 7**) therethrough. A proximal end of drive member **120** is operably connected to actuation rod **80** such that translation of actuation rod **80** correspondingly translates drive member **120.** Anvil channel **111** may be divided into a first channel **111a** configured for passage of drive member **120** therethrough and a second channel **111b** for passage of translatable tip **150** therethrough. Cartridge assembly **108** includes a plurality of staples **130** and a plurality of pushers **132** therein. Pushers **132** may be translated by a sled **122** (**FIG. 6A**) toward anvil cartridge **106** to push the plurality of staples **130** into contact with a corresponding plurality of anvil pockets **134** in anvil cartridge **106.** Pressure between staples **130** and anvil pockets **134** deforms staples **130** into a suitable configuration for stapling tissue.

As seen in **FIG. 6A****,** anvil channel **111** houses a translation rod **154** for longitudinally translating translatable tip **150** between the extended and retracted states upon a sliding of translation switch **32.** A proximal end of translation rod **154** is operably connected to translation switch **32.** A distal end of translation rod **154** is operably connected to translatable tip **150.** Translation rod **154** may have multiple segments in embodiments of surgical stapling device **10** in which end effector **100** is releasably attached to elongated shaft **18.** Cartridge channel **112** houses sled **122,** as will be discussed in greater detail hereinbelow.

**FIGS. 6A-8** illustrate transitioning of tool assembly **104.** **FIG. 6A** illustrates a typical state of tool assembly **104** at a beginning of a surgical procedure. Tool assembly **104** is in a closed position and translatable tip **150** is retracted within anvil assembly **106** for tool assembly **104** to have a minimal configuration. A minimal configuration of tool assembly **104** assists in movement of tool assembly **104** through a surgical site. At the surgical site, rotation knob **14** and articulation knob **16** may be each rotated to a degree necessary to suitably position tool assembly **104** for grasping tissue.

Tool assembly **104** is transitioned from the open position to the closed position by partially translating drive member **120** distally through anvil channels **111, 112.** In the grasping mode, drive member **120** cannot translate far enough distally to push sled **122** distally. In the firing mode, drive member **120** is translated far enough distally to push sled **122** distally. Tool assembly **104** is transitioned from the closed position to the open position by translating drive member **120** proximally.

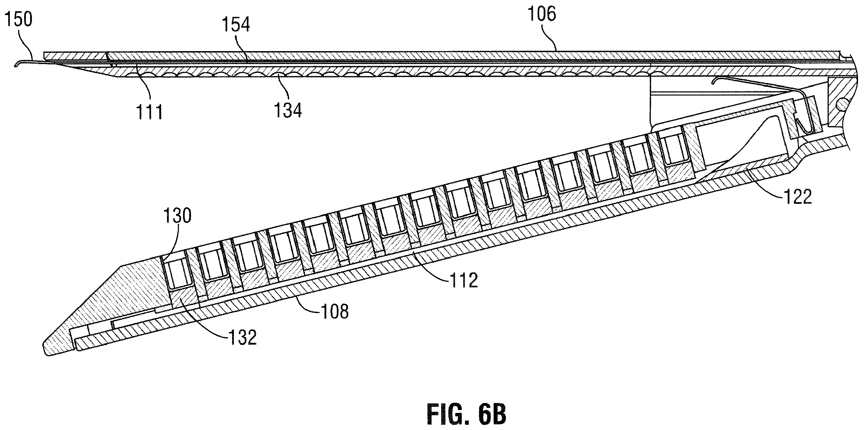

As seen in **FIG. 6B****,** tool assembly **104** may be transitioned to the open position to receive tissue therebetween. To assist in manipulating tissue, translatable tip **150** may be extended from anvil channel **111.** Once tissue is between anvil assembly **106** and cartridge assembly **108,** tool assembly **104** can be transitioned to the closed position to grasp the tissue or fire staples therethrough. Translatable tip **150** may be retracted or may remain extended when tool assembly **104** is in the closed position.

As seen in **FIGS. 7-8****,** staples can be fired when tool assembly **104** is in the closed position. First, surgical stapling device **10** is switched from the grasping mode to the firing mode by sliding actuator button **28** distally. Movable handle **22** is repeatedly pulled to the first position and released to the second position, each time incrementally advancing drive member **120** distally through channels **111, 112.** Drive member **120** pushes sled **122** distally through cartridge assembly **108.** Sled **122** sequentially engages pushers **132,** which push staples **130** into anvil pockets **134.** At any time surgical stapling device **10** is in the grasping mode, such as when stapling is complete, retraction handles **26** may be pulled proximally to return surgical stapling device **10** to the grasping mode. Then surgical stapling device **10** may be removed from the surgical site. To assist in removal, translatable tip **150** may be retracted within anvil channel **111** and tool assembly may be transitioned to the closed position to for a minimal configuration of tool assembly **104.**

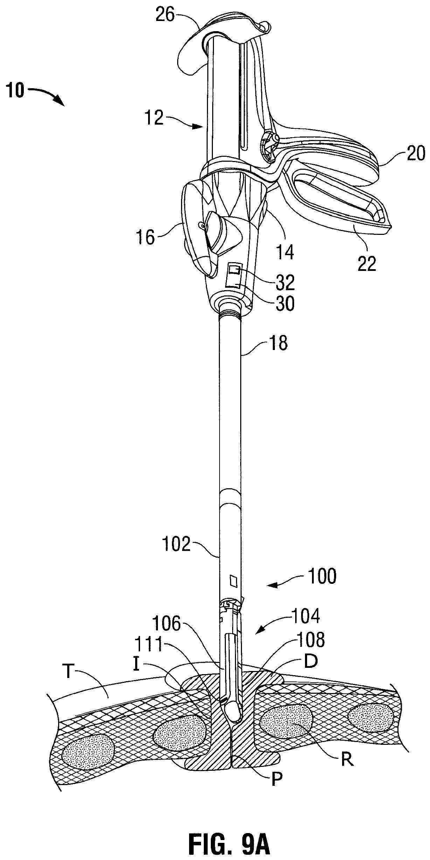

**FIGS. 9A-9C** illustrate a method of use of surgical stapling device **10.** Although the method is described with respect to a thoracic procedure, it should be understood that substantially similar methods may also be used for endoscopic, laparoscopic, and other minimally invasive procedures. As seen in **FIG. 9A****,** an incision **I** is created in a tissue **T** in an intercostal space between ribs **R.** An access device **D** is inserted through incision **I** to facilitate creating a substantially fluid-tight seal about incision **I.** Access device **D** may be formed of a compliant foam or plastic that conforms to tissue **T.** Alternatively, access device **D** may be formed of a substantially firm material to provide a force that spread ribs **R** apart to create a larger working area for the thoracic procedure. Access device could also be configured to stretch a flexible membrane to stretch the incision. Access device **D** has at least one port **P** extending therethrough for receipt of surgical stapling device **10** therethrough. During insertion of surgical stapling device **10** through portion **P,** surgical access device is in the grasping mode. Movable handle **22** is pulled to the first position to close tool assembly **104.** Switch **32** is slid proximally to retract tip **150.**

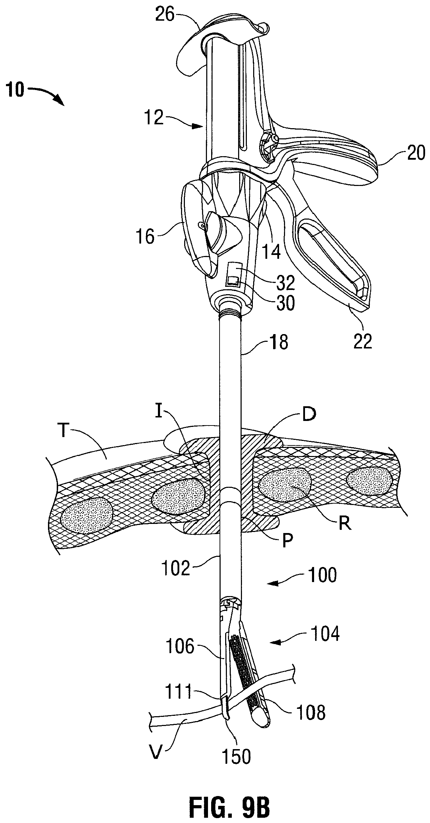

As seen in **FIG. 9B****,** surgical access device **10** is inserted through port **P.** End effector **100** passes completely through port **P.** Handle assembly **12** remains proximal to port **P.** A portion of elongated member **18** is within port **P,** and material defining port **P** forms a substantially fluid-tight seal with the portion of elongated member **18** therein. Sliding switch **32** distally extends tip **150** to manipulate a vessel **V** underneath tissue **T.** Movable handle **22** may be pulled and released as necessary for tool assembly **104** to grasp, manipulate, and maneuver through tissue. Rotating actuator handle **16** and rotation knob **14** may assist in maneuvering end effector **100** through tissue.

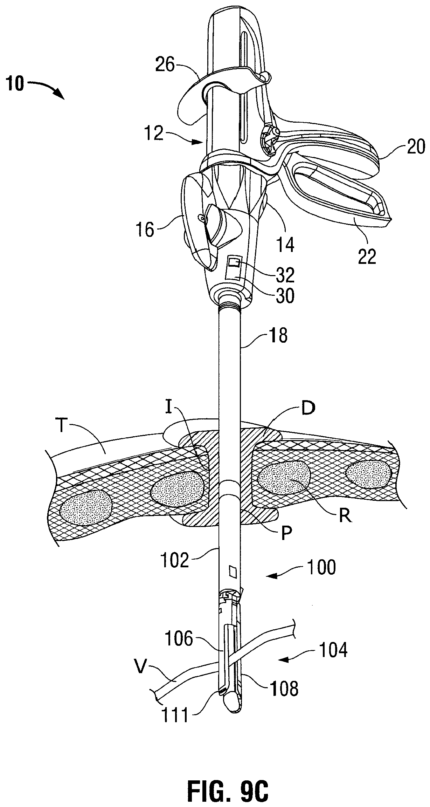

As seen in **FIG. 9C****,** once vessel **V** has been grasped within tool assembly **104,** staples **130** may be fired through vessel **V.** Actuation button **28** is slid distally to switch surgical device **10** to the firing mode, as seen in **FIG. 3D****.** Movable handle **22** is repeatedly pulled and released to incrementally fire staples **130.** Each pulling of movable handle **22** incrementally moves retraction handles **26** distally. When firing is complete, retraction handles **26** are pulled proximally to return surgical stapling device **10** to the grasping mode and release vessel **V** from tool assembly **104.** Surgical stapling device **10** may then be removed from the surgical site by reversing the insertion steps described hereinabove.

**FIG. 10** illustrates another embodiment of an anvil assembly of a surgical stapling device according to the present disclosure and is designated as **206.** Anvil assembly **206** may replace or be used instead of anvil assembly **106.** Anvil assembly **206** is similar to anvil assembly **106** and thus will only be discussed in detail herein to the extent necessary to identify differences in construction and operation thereof. Anvil assembly **206** includes a pneumatic tip **250.** Pneumatic tip **250** is compliant. Pneumatic tip **250** has a rolled configuration in a retracted state and an unrolled configuration in an extended state. Pneumatic tip **250** is connected to a distal end of translation rod **154.** Pneumatic tip **250** and translation rod **154** are hollow and adapted for receipt of a pressurized fluid, such as pressurized CO₂, therethrough. A proximal end of translation rod **154** is in fluid communication with any appropriate pneumatic supply, such as a pump or a compressed gas cartridge, for extending and retracting pneumatic tip **250.** Pneumatic tip **250** may be biased to the retracted state, such that fluid pressure extends pneumatic tip **250** and removal of fluid pressure retracts pneumatic tip **250.**

**FIG. 11** illustrates another embodiment of a tool assembly of a surgical stapling device according to the present disclosure and is generally designated as **304.** Tool assembly **304** may replace or be used instead of tool assembly **104.** Tool assembly **304** is similar to tool assembly **104** and thus will only be discussed in detail herein to the extent necessary to identify differences in construction and operation thereof. Tool assembly **304** includes an anvil assembly **306** and a cartridge assembly **308** that are pivotably movable with respect to each other between an open position and a closed position. Anvil assembly **306** has a kinked tip **350** for manipulating tissue. When tool assembly **304** is in the closed position, kinked tip **350** and a distal end of cartridge assembly **308** are substantially parallel and define a tissue engagement plane therebetween. Cartridge assembly **308** is longitudinally translatable between a retracted state and an extended state upon a sliding of translation switch **32.** As anvil assembly **306** remains in place as cartridge assembly is retracted, retracting cartridge **308** widens the engagement plane, thus allowing kinked tip **350** to more easily manipulate larger tissue. In an alternate embodiment, anvil assembly **306** may be longitudinally translatable between a retracted state and an extended state as cartridge assembly **308** remains stationary. Embodiments are also conceived in which a distal tip of cartridge assembly **308** is distal to a distal tip of anvil assembly **306,** in which case cartridge assembly **308** may be extendable or anvil assembly **306** may be retractable to widen the engagement plane.

**FIG. 12** illustrates another embodiment of an end effector of a surgical stapling device according to the present disclosure and is generally designated as **400.** End effector **400** may replace or be used instead of end effector **100.** End effector **400** is similar to end effector **100** and thus will only be discussed in detail herein to the extent necessary to identify differences in construction and operation thereof. End effector **400** includes a body portion **402** and a tool assembly **404.** Tool assembly **404** includes an anvil assembly **406** and cartridge assembly **408** that are pivotably movable with respect to each other. Body portion **402** includes an extendable portion **402a** that is extendable and retractable along the longitudinal axis defined by elongated member **18** to simultaneously extend or retract both anvil assembly **406** and cartridge assembly **408,** thereby assisting the user in manipulating tissue. Extension and retraction of extendable portion **402a** are actuated by sliding translation switch **32** distally and proximally, respectively.

Anvil assembly **406** has an LED tip **460** for use as a visual aid. Alternatively, cartridge assembly **408** could have an LED tip. LED tip **460** may be curved and/or tapered to assist in manipulating tissue. In another embodiment, at least one of anvil assembly **406** and cartridge assembly **408** has a sensor tip. The sensor tip may be used to sense a tissue condition. The sensor tip may be in wired or wireless communication with a processor for processing sensed information about the tissue condition.

Anvil assembly **406** includes a distal end of a tube **470.** Tube **470** may serve many functions, such as evacuation of material within the surgical site, irrigation of the surgical site, and absorption of material from the surgical site. Tube **470** extends proximally from anvil assembly **406** through end effector **400** and further through elongated member **18.** A proximal end of tube **470** may be operably connected to a mechanism, such as a vacuum source or an irrigation source, for fluid communication with the surgical site.

It will be understood that various modifications may be made to the embodiments disclosed herein. For example, it is envisioned that the surgical stapling device disclosed may be used in association with other surgical devices, e.g., clip appliers, dissectors, electrosurgical sealing devices, etc. Further, the device may also include tool assemblies other than staplers or those devices which eject a fastener, e.g., sealing devices (electrosurgical and non-electrosurgical), etc. Therefore, the above description should not be construed as limiting, but merely as exemplifications of preferred embodiments. Those skilled in the art will envision other modifications within the scope and spirit of the claims appended hereto.

The invention may be described by reference to the following numbered paragraphs:-
1. A surgical device comprising:
   a handle assembly;
   an elongated member extending distally from the handle assembly, the elongated member defining a longitudinal axis;
   a tool assembly mounted to a distal end of the elongated member, the tool assembly having a cartridge assembly having a plurality of staples supported therein and an anvil assembly, at least one of the anvil assembly and the cartridge assembly being movable in relation to each other between open and closed positions, at least one of the anvil assembly and the cartridge assembly having at least a portion thereof being configured for transitioning between a longitudinally extended state and a longitudinally retracted state; and
   a transitioning mechanism configured to transition at least one of the anvil assembly and the cartridge assembly between the extended and retracted states.
2. The surgical device according to paragraph 1, wherein at least one of the cartridge assembly and the anvil assembly includes a tongue that transitions between an extended state and a retracted state.
3. The surgical device according to paragraph 2, wherein the transitioning mechanism pneumatically transitions the tongue.
4. The surgical device according to paragraph 2, wherein the transitioning mechanism includes a rod for transitioning the tongue.
5. The surgical device according to paragraph 2, wherein the tongue has an arcuate configuration.
6. The surgical device according to paragraph 2, wherein the tongue transitions out from and into at least one of the cartridge assembly and the anvil assembly.
7. The surgical device according to paragraph 1, wherein at least one of the cartridge assembly and the anvil assembly includes a visual aid disposed thereon.
8. The surgical device according to paragraph 2, wherein the tongue is composed of shape memory material and returns to a shape memorized position in the extended state.
9. The surgical device according to paragraph 1, wherein at least one of the cartridge assembly and the anvil assembly includes a sensor disposed thereon for sensing a tissue condition.
10. The surgical device according to paragraph 1, wherein at least one of the cartridge assembly and the anvil assembly distal to an engagement plane transitions between the expanded state and the contracted state.
11. The surgical device according to paragraph 10, wherein at least one of the cartridge assembly and the anvil assembly proximal to an engagement plane remains stationary as at least one of the cartridge assembly and the anvil assembly distal to an engagement plane transitions between the expanded state and the contracted state.
12. The surgical device according to paragraph 1, wherein at least one of the cartridge assembly and the anvil assembly proximal to an engagement plane transitions between the expanded state and the contracted state.
13. The surgical device according to paragraph 12, wherein at least one of the cartridge assembly and the anvil assembly distal to an engagement plane remains stationary as at least one of the cartridge assembly and the anvil assembly proximal to an engagement plane transitions between the expanded state and the contracted state.
14. The surgical device according to paragraph 1, wherein at least one of the cartridge assembly and the anvil assembly includes a tube configured for passage of material therethrough.
15. The surgical device according to paragraph 1, wherein the cartridge assembly and the anvil assembly move between the open and closed positions in response to actuation of a movable handle of the handle assembly.
16. The surgical device according to paragraph 1, wherein the cartridge assembly fires the plurality of staples in response to actuation of a movable handle of the handle assembly.
17. The surgical device according to paragraph 1, further including a mode selection mechanism configured to alternate the surgical device between a first mode of operation and a second mode of operation, the mode selection mechanism including a switch for selecting between the first mode of operation, in which the cartridge assembly and anvil assembly are movable back and forth between the open and closed positions, and the second mode of operation, in which the cartridge assembly and anvil assembly are locked in the closed position for clamping tissue therebetween and firing the plurality of staples.
18. A method of stapling tissue, comprising the steps of:
   inserting a surgical stapling device including an end effector having an extendable tip through an incision in tissue;
   extending the extendable tip;
   manipulating tissue with the extendable tip;
   grasping tissue within the end effector;
   switching the surgical stapling device from a grasping mode to a firing mode; and
   firing staples from the end effector through tissue.
19. The method according to paragraph 18, further comprising the step of pulling a retraction handle of the surgical stapling device to release tissue from the end effector and return the surgical stapling device from the firing mode to the grasping mode.
20. The method according to paragraph 18, wherein the extendable tip is a tongue movable out from and into an anvil assembly or a cartridge assembly of the end effector.
The invention may also be described by reference to the following numbered paragraphs:-
1. A surgical device comprising:
   a handle assembly;
   an elongated member extending distally from the handle assembly, the elongated member defining a longitudinal axis;
   a tool assembly mounted to a distal end of the elongated member, the tool assembly having a cartridge assembly having a plurality of staples supported therein and an anvil assembly, at least one of the anvil assembly and the cartridge assembly being movable in relation to each other between open and closed positions, at least one of the anvil assembly and the cartridge assembly having at least a portion thereof being configured for transitioning between a longitudinally extended state and a longitudinally retracted state; and
   a transitioning mechanism configured to transition at least one of the anvil assembly and the cartridge assembly between the extended and retracted states.
2. The surgical device according to paragraph 1, wherein at least one of the cartridge assembly and the anvil assembly includes a tongue that transitions between an extended state and a retracted state.
3. The surgical device according to paragraph 2, wherein the transitioning mechanism pneumatically transitions the tongue.
4. The surgical device according to paragraph 2 or paragraph 3, wherein the transitioning mechanism includes a rod for transitioning the tongue.
5. The surgical device according to any one of paragraphs 2 to 4, wherein the tongue has an arcuate configuration.
6. The surgical device according to any one of paragraphs 2 to 5, wherein the tongue transitions out from and into at least one of the cartridge assembly and the anvil assembly.
7. The surgical device according to any preceding paragraph, wherein at least one of the cartridge assembly and the anvil assembly includes a visual aid disposed thereon.
8. The surgical device according to any one of paragraphs 2 to 7, wherein the tongue is composed of shape memory material and returns to a shape memorized position in the extended state.
9. The surgical device according to any preceding paragraph, wherein at least one of the cartridge assembly and the anvil assembly includes a sensor disposed thereon for sensing a tissue condition.
10. The surgical device according to any preceding paragraph, wherein at least one of the cartridge assembly and the anvil assembly distal to an engagement plane transitions between the expanded state and the contracted state.
11. The surgical device according to paragraph 10, wherein at least one of the cartridge assembly and the anvil assembly proximal to an engagement plane remains stationary as at least one of the cartridge assembly and the anvil assembly distal to an engagement plane transitions between the expanded state and the contracted state.
12. The surgical device according to any preceding paragraph, wherein at least one of the cartridge assembly and the anvil assembly proximal to an engagement plane transitions between the expanded state and the contracted state.
13. The surgical device according to paragraph 12, wherein at least one of the cartridge assembly and the anvil assembly distal to an engagement plane remains stationary as at least one of the cartridge assembly and the anvil assembly proximal to an engagement plane transitions between the expanded state and the contracted state.
14. The surgical device according to any preceding paragraph, wherein at least one of the cartridge assembly and the anvil assembly includes a tube configured for passage of material therethrough.
15. The surgical device according to any preceding paragraph, wherein the cartridge assembly and the anvil assembly move between the open and closed positions in response to actuation of a movable handle of the handle assembly.
16. The surgical device according to any preceding paragraph, wherein the cartridge assembly fires the plurality of staples in response to actuation of a movable handle of the handle assembly.
17. The surgical device according to any preceding paragraph, further including a mode selection mechanism configured to alternate the surgical device between a first mode of operation and a second mode of operation, the mode selection mechanism including a switch for selecting between the first mode of operation, in which the cartridge assembly and anvil assembly are movable back and forth between the open and closed positions, and the second mode of operation, in which the cartridge assembly and anvil assembly are locked in the closed position for clamping tissue therebetween and firing the plurality of staples.

## Claims

1. A surgical device comprising:
a handle assembly (12);
an elongated member (18) extending distally from the handle assembly, the elongated member defining a longitudinal axis;
a tool assembly (104) mounted to a distal end of the elongated member, the tool assembly having a cartridge assembly (108) having a plurality of staples (130) supported therein and an anvil assembly (206), at least one of the anvil assembly and the cartridge assembly being movable in relation to each other between open and closed positions, the anvil assembly having a portion thereof being configured for transitioning between a longitudinally extended state and a longitudinally retracted state; and
a transitioning mechanism configured to transition the anvil assembly (206) between the extended and retracted states,
**characterized in that**:
said portion configured for transitioning between a longitudinally extended state and a longitudinally retracted state comprises a compliant pneumatic tip (250) having a rolled configuration in the retracted state and an unrolled configuration in the extended state, the pneumatic tip being biased to the retracted state; and
said transitioning mechanism comprises a translation rod (154), the pneumatic tip (250) and the translation rod being hollow for receipt of a pressurized fluid therethrough, whereby application of a fluid pressure through the hollow translation rod (154) extends the pneumatic tip (250) and removal of the fluid pressure retracts the pneumatic tip (250).

2. The surgical device according to claim 1, wherein the cartridge assembly (108) is configured to fire the plurality of staples (130) in response to actuation of a movable handle (22) of the handle assembly (12).

3. The surgical device according to claim 1 or 2, further including a switch (28) for selecting between a first mode of operation, in which the cartridge assembly (108) and anvil assembly (106) are movable back and forth between the open and closed positions, and a second mode of operation, in which the cartridge assembly (108) and anvil assembly (106) are locked in the closed position for clamping tissue therebetween and firing the plurality of staples (130).
